# EUROPEAN PATENT APPLICATION

(11) **EP 4 397 316 A1**
(43) Date of publication of application: **10.07.2024**
(21) Application number: 22864475.3
(22) Date of filing: 29.08.2022
(51) Int. Cl.: A61K 39/39, A61K 9/113, A61K 9/14, A61K 47/34, A61K 47/36, A61P 35/00, A61P 37/04

(54) **COMPOSITION FOR ENHANCING IMMUNOGENICITY**

(30) Priority: 30.08.2021 JP 2021139684
(71) Applicant: Toray Industries, Inc., Tokyo 103-8666 (JP)
(72) Inventor: SHIBAHARA Kyoko, Kamakura-shi, Kanagawa 248-8555 (JP); OKANO Fumiyoshi, Kamakura-shi, Kanagawa 248-8555 (JP); PARK Joonsik, Kamakura-shi, Kanagawa 248-8555 (JP)
(74) Representative: Mewburn Ellis LLP
(86) International application number: PCT/JP2022/032352
(87) International publication number: WO 2023/032892

(57) **Abstract**

Provided is a composition for enhancing immunogenicity, having potent immuno-stimulating capacity. A composition for enhancing immunogenicity, containing, as an active ingredient, a mixture and an immuno-stimulating factor, wherein the mixture comprises a particle comprising an amphiphilic polymer in which a hydrophobic segment is poly(hydroxy acid) and a hydrophilic segment is β-glucan, and an antigen.

## Description

### Technical Field

The present invention relates to a composition for enhancing immunogenicity, containing, as an active ingredient, a mixture of a particle and an immuno-stimulating factor, wherein the particle comprises an amphiphilic polymer in which a hydrophobic segment is poly(hydroxy acid) and a hydrophilic segment is β-glucan, and an antigen.

### Background Art

Since administration of antigens alone is insufficient for inducing immune responses to antigens sufficiently, adjuvants (immuno-stimulating factors) are used in combination. However, substances reported to have a high adjuvant effect are not approved for use in humans due to safety issues. While limited adjuvants including aluminum hydroxide and MF59 are used for medicaments, there is a demand for development of adjuvants not only ensuring safety, but also capable of inducing immune responses more potently.

In order to solve the above problems, microparticle adjuvants are progressively under development. Formation of particles allows for enclosure of antigens and immuno-stimulator, and thus an enhancement in safety and an enhancement in delivery efficiency to target cells are expected. There have been proposed microparticle adjuvants with diverse materials such as fatty acids, biodegradable polymers, and viral proteins (see Non Patent Literature 1 and 2.).

In recent years, there has been a reported technique relating to an antigen-adjuvant microparticle complex, wherein an antigen is enclosed in an adjuvant microparticle consisting of an amphiphilic polymer in which a hydrophobic segment is poly(hydroxy acid) and a hydrophilic segment is polysaccharide including β-glucan (see Patent Literature 1 and 2). This technique has successfully induced a high immune response to an antigen by a small amount of the antigen and a small number of administrations.

However, to date, an effective adjuvant having much better performances than conventional adjuvants has not been realized by techniques using existing microparticles, although development thereof has been hoped.

### Citation List

### Patent Literature

Patent Literature 1: WO2010/098432
Patent Literature 2: WO2015/053354

### Non Patent Literature

Non Patent Literature 1: Immunology, vol. 117, 2006, pages 77 to 88
Non Patent Literature 2: Nature Materials, vol. 10, 2011, pages 243 to 251

### Summary of Invention

### Technical Problem

An object of the present invention is to provide a composition for enhancing immunogenicity, having potent immuno-stimulating capacity, by enhancing immuno-stimulating action of a particle composed of an amphiphilic polymer in which a hydrophobic segment is poly(hydroxy acid) and a hydrophilic segment is β-glucan.

### Solution to Problem

In order to overcome the above problems, the present inventor has found that a composition for enhancing immunogenicity, containing, as an active ingredient, a mixture of a particle and an immuno-stimulating factor, wherein the particle comprises an amphiphilic polymer in which a hydrophobic segment is poly(hydroxy acid) and a hydrophilic segment is β-glucan, and an antigen is effective, and thus has completed the present invention.

In other words, the present invention has the constitution as shown in the following (1) to (15).
(1) A composition for enhancing immunogenicity, containing, as an active ingredient, a mixture of a particle and an immuno-stimulating factor, wherein the particle comprises an amphiphilic polymer in which a hydrophobic segment is poly(hydroxy acid) and a hydrophilic segment is β-glucan, and an antigen.
(2) The composition for enhancing immunogenicity according to (1), wherein the mixture comprises each of the particle and the immuno-stimulating factor in an independent state.
(3) The composition for enhancing immunogenicity according to (1) or (2), wherein the β-glucan is a polymer of glucose linked by one or more β-1,3 bonds and/or one or more β-1,6 bonds.
(4) The composition for enhancing immunogenicity according to any of (1) to (3), wherein the β-glucan is black yeast glucan, curdlan, pachyman, laminaran, lichenan, schizophyllan, lentinan, scleroglucan or pachymaran.
(5) The composition for enhancing immunogenicity according to any of (1) to (4), wherein the poly(hydroxy acid) is poly(lactic-co-glycolic acid), polylactic acid or polyglycolic acid.
(6) The composition for enhancing immunogenicity according to any of (1) to (5), wherein the immuno-stimulating factor is a ligand or agonist binding to a Toll-like receptor (TLR), a NOD-like receptor (NLR), a RIG-like receptor or a C-type lectin receptor (CLR), or a stimulator of interferon gene (STING).
(7) The composition for enhancing immunogenicity according to (6), wherein the ligand or agonist binding to the Toll-like receptor (TLR) is a ligand or agonist binding to TLR2, TLR3, TLR4, TLR5, TLR7, TLR8, TLR9 or TLR11.
(8) The composition for enhancing immunogenicity according to (6) or (7), wherein the ligand or agonist binding to the Toll-like receptor (TLR) is any of the following (i) to (vii):
   (i) a ligand or agonist binding to TLR2 selected from the group consisting of peptidoglycan, lipoprotein, lipopolysaccharide and zymosan
   (ii) a ligand or agonist binding to TLR3 selected from the group consisting of poly(I:C) and poly(A:U)
   (iii) a ligand or agonist binding to TLR4 selected from the group consisting of lipopolysaccharide (LPS), HSP60, RS09 and MPLA
   (iv) flagellin as a ligand or agonist binding to TLR5
   (v) a ligand or agonist binding to TLR7 or 8 selected from the group consisting of an imidazoquinoline compound (imidazoquinoline species) and single-strand RNA
   (vi) a ligand or agonist binding to TLR9 selected from the group consisting of bacterial DNA, unmethylated CpG DNA, hemozorin, ODN1585, ODN1668, and ODN1826, and
   (vii) a ligand or agonist binding to TLR11 selected from the group consisting of profilin and uropathogenic bacteria.
(9) The composition for enhancing immunogenicity according to any of (1) to (8), wherein a number-average molecular weight of the amphiphilic polymer is 500 to 100,000 (molar ratio).
(10) The composition for enhancing immunogenicity according to any of (1) to (9), wherein an average particle size of the particle is 0.1 to 50 µm.
(11) A medicine containing the composition for enhancing immunogenicity according to any of (1) to (10), as an active ingredient.
(12) A vaccine containing the composition for enhancing immunogenicity according to any of (1) to (10), as an active ingredient.
(13) A vaccine for treatment and/or prevention of cancer, containing the composition for enhancing immunogenicity according to any of (1) to (10), as an active ingredient.
(14) A method for enhancing immunogenicity, comprising administering the composition for enhancing immunogenicity according to any of (1) to (10), to a subject.
(15) A method for treating and/or preventing cancer, comprising administering the composition for enhancing immunogenicity according to any of (1) to (10), the medicine according to (11), or the vaccine according to (12) or (13), to a subject.

The present specification encompasses the disclosure of Japanese Patent Application No. 2021-139684 which is the basis of the priority claim of the present application.

### Advantageous Effects of Invention

The present invention provides a composition for enhancing immunogenicity, which can allow for immuno-stimulating more potently than conventional ones.

### Brief Description of Drawings

[Figure 1] Figure 1 represents the results of GPC measurement of black yeast glucan-NH₂ and black yeast glucan-PLGA.
[Figure 2] Figure 2 represents the results of ¹H-NMR measurement of black yeast glucan-PLGA.
[Figure 3] Figure 3 represents the results of dynamic light scattering (DLS) measurement of an OVA-containing black yeast glucan particle (1).

### Description of Embodiments

The term "composition for enhancing immunogenicity" in the present invention refers to a composition that can enhance an immune response to an antigen *in vivo.* The composition for enhancing immunogenicity of the present invention contains, as an active ingredient, a mixture of a particle and an immuno-stimulating factor, wherein the particle comprises an amphiphilic polymer in which a hydrophobic segment is poly(hydroxy acid) and a hydrophilic segment is β-glucan, and an antigen. The type of the immune response generated by the composition for enhancing immunogenicity is not particularly limited. Generally, it is known that there are Th1-type immune response and Th2-type immune response as the types of immune response, and that preferentially generated immune response among them depends on the types of an antigen, an immuno-stimulating factor, an administration site, and an administration method. The present invention may induce both Th1-type and Th2-type immune responses.

The amphiphilic polymer constituting a particle together with an antigen is described. The term "amphiphilic" means having both properties of hydrophilicity and hydrophobicity. When the solubility in water of a certain portion (segment) is higher than that of other portions, the certain portion (segment) is referred to as being hydrophilic. It is desirable that the hydrophilic portion is soluble in water, but may be hardly soluble in water as long as it has higher solubility in water compared to other portions. When the solubility in water of a certain portion (segment) is lower than that of other portion, the certain portion (segment) is referred to as being hydrophobic. It is desirable that the hydrophobic portion is insoluble in water, but may be soluble in water as long as it has lower solubility in water as compared with other portions.

The term "amphiphilic polymer" means a polymer having the above amphiphilicity as the whole molecule. The amphiphilic "polymer" indicates an amphiphilic molecule having a hydrophilic segment or a hydrophobic segment, or both thereof with a molecular structure constituted from a repeated structure of a minimum unit (monomer). The structure of the amphiphilic polymer in the present invention is not particularly limited, and specific examples thereof include: a straight-chained block-type polymer in which β-glucan and poly(hydroxy acid) are connected, a branched polymer having branches, comprising a plurality of β-glucans or poly(hydroxy acids); a graft-type polymer comprising a main chain of β-glucan and a side chain of poly(hydroxy acid); and a graft-type polymer comprising a main chain of poly(hydroxy acid) and a side chain of β-glucan; and preferably, a straight-chained block-type polymer in which β-glucan and poly(hydroxy acid) are connected.

The present invention is characterized in that the hydrophilic segment of the amphiphilic polymer is β-glucan. The glucan refers to glucose-containing polysaccharides, and the β-glucan refers to glucan containing one or more β-bonds between glucose subunits. In other words, the β-glucan for use in the present invention contains a β-bond, and may contain only a β-bond. The β-glucan for use in the present invention may be branched or linear. Examples of preferable β-glucan include β-glucan containing one or more β-1,3 bonds and/or one or more β-1,6 bonds, or β-glucan containing one or more β-1,2 bonds and/or a β-1,4 bond, and β-glucan containing one or more β-1,3 bonds and/or one or more β-1,6 bonds is more preferable, and β-glucan containing one or more β-1,3 bonds is further preferable. Specific examples of the β-glucan containing one or more β-1,3 bonds include curdlan, pachyman, laminaran, lichenan, schizophyllan, lentinan, scleroglucan, black yeast glucan (preferably, black yeast-derived β-1,3 glucan or β-1,6 glucan) or pachymaran, and preferably include curdlan, pachyman, laminaran, schizophyllan, scleroglucan, black yeast glucan or pachymaran.

Examples of linear β-glucan containing one or more β-1,3 bonds include β-glucan mainly containing a β-1,3 bond (for example, curdlan or pachyman), or β-glucan containing a β-1,3 bond and a β-bond other than a β-1,3 bond (for example, laminaran or lichenan).

Examples of branched β-glucan containing one or more β-1,3 bonds include β-glucan containing a β-1,3 bond and a β-1,6 bond (for example, schizophyllan or lentinan, scleroglucan, black yeast glucan).

The β-glucan for use in the present invention may be derivatized β-glucan. Examples of such derivatization include addition reaction of a carboxymethyl group, or oxidative cleavage reaction. Examples of the derivatized β-glucan include carboxymethyl curdlan obtained by adding a carboxymethyl group to curdlan, or pachymaran obtained by cleaving pachyman.

The number-average molecular weight of β-glucan is not particularly limited, and is preferably 500 to 100,000, more preferably 500 to 50,000, further preferably 1,000 to 10,000, for example, 1,000 to 8,000, 1,000 to 6,000, 1,000 to 4,000, 1,000 to 3,000 or 1,000 to 2,500. The number-average molecular weight is an average molecular weight calculated by a method in which weighting for the size of a molecule is not considered (simple average). The number-average molecular weight of β-glucan may be determined by gel permeation chromatography (GPC).

The present invention is characterized in that the hydrophobic segment of the amphiphilic polymer is poly(hydroxy acid). The poly(hydroxy acid) is not particularly limited, and is preferably a biocompatible polymer without remarkable adverse effect upon administration into an organism. The biocompatibility herein refers to having an LD50 of 2,000 mg/kg or more when the polymer is orally administered to a rat. The poly(hydroxy acid) may be a copolymer of a plurality of types of hydroxy acids, and is preferably a polymer of two or less types of hydroxy acids.

Specific examples of the poly(hydroxy acid) preferably include polyglycolic acid, polylactic acid, poly(2-hydroxybutyric acid), poly(2-hydroxyvaleric acid), poly(2-hydroxycaproic acid), poly(2-hydroxycapric acid), poly(malic acid), or any derivative and copolymer of such polymer compounds, and preferably, poly(lactic-co-glycolic acid), polylactic acid or polyglycolic acid, and more preferably, poly(lactic-co-glycolic acid). In a case where the poly(hydroxy acid) is poly(lactic-co-glycolic acid), the compositional ratio (lactic acid/glycolic acid) (mol/mol) of the poly(lactic-co-glycolic acid) is not particularly limited as long as the problems of the present invention are achieved, and is preferably 99/1 to 1/99, more preferably 80/20 to 20/80, for example, 60/40 to 40/60, or 50/50.

The number-average molecular weight of the poly(hydroxy acid) portion in the amphiphilic polymer in the present invention is not particularly limited, and is preferably 500 to 1,000,000, more preferably 500 to 100,000, for example, 1,000 to 90,000, 3,000 to 80,000, 5,000 to 70,000, or 6,000 to 60,000, further preferably 7,000 to 50,000, for example, 7,000 to 40,000, 7,000 to 30,000, 7,000 to 20,000, 7,000 to 15,000, 7,000 to 13,000, or 7,000 to 11,500. The number-average molecular weight of poly(hydroxy acid) can be determined from the difference between the number-average molecular weight of the amphiphilic polymer, in which a hydrophobic segment is poly(hydroxy acid) and a hydrophilic segment is β-glucan, and the number-average molecular weight of β-glucan.

The number-average molecular weight of the amphiphilic polymer constituting the particle is not particularly limited, and is preferably 1,000 to 1,000,000, more preferably 1,000 to 100,000, further preferably 9,000 to 50,000, for example, 9,000 to 40,000, 9,000 to 30,000, 9,000 to 20,000, 9,000 to 15,000, or 9,000 to 13,000. The number-average molecular weight of the amphiphilic polymer can be determined by gel permeation chromatography (GPC).

The amphiphilic polymer may be produced by a known method, and specific examples of the method include a production method comprising adding poly(hydroxy acid) to β-glucan and performing a condensation reaction, or a production method comprising adding a hydroxy acid activated monomer to β-glucan and performing a polymerization reaction.

In a case where the amphiphilic polymer is a straight-chained block-type polymer in which β-glucan and poly(hydroxy acid) are connected, the polymer may be produced by a known method, and specific examples of the method include a production method comprising performing a condensation reaction of a poly(hydroxy acid) copolymer at a reducing terminal of β-glucan of the amphiphilic polymer using an activator of a terminal functional group [Macromol. Rapid Commun., 31, p.1664-1684 (2010)].

In a case where the amphiphilic polymer is a graft-type polymer comprising a main chain of β-glucan and a side chain of poly(hydroxy acid), the polymer may be produced according to the following method (1), (2), or (3).

(1) A method of producing a graft-type polymer, in which a poly(hydroxy acid) is introduced by adding a hydroxy acid activated monomer to β-glucan to perform polymerization reaction in the presence of a tin catalyst [Macromolecules, 31, p. 1032-1039 (1998)].
(2) A method of producing a graft-type polymer, in which a graft chain of poly(hydroxy acid) is introduced by activating some unprotected hydroxyl groups of β-glucan in which most hydroxyl groups are protected with substituents, with a base, then adding a hydroxy acid activated monomer thereto, and protect groups are finally removed [Polymer, 44, p. 3927-3933, (2003)].
(3) A method of producing a graft-type polymer, in which the condensation reaction of a copolymer of poly(hydroxy acid) to β-glucan is performed by using a dehydrating agent and/or an activator of a functional group [Macromolecules, 33, p. 3680-3685 (2000)].

The amphiphilic polymer is preferably water-insoluble as a whole in order to keep immuno-stimulating action for a long period and not to be rapidly excreted *in vivo.* The term "water-insoluble" herein refers to having a solubility in water of 1 g (amphiphilic polymer)/100 ml (water) or less.

The amphiphilic polymer may be a modified amphiphilic polymer to which a desired function is imparted by modification with a functional compound. As a specific example, the amphiphilic polymer may be a modified amphiphilic polymer in which an additional immuno-stimulating factor, other than the immuno-stimulating factor as an active ingredient of the composition for enhancing immunogenicity of the present invention, is bound to the amphiphilic polymer by a known method in order to enhance immuno-stimulating action.

The antigen constituting the particle together with the amphiphilic polymer is described. The term "antigen" in the present invention refers to a substance which triggers immunity *in vivo* and thus can be utilized as a vaccine for treatment and/or prevention of disease. An immune response triggered by the antigen can be enhanced by using, as an active ingredient, the particle comprising the antigen and the amphiphilic polymer of the present invention.

Examples of the antigen include peptide, protein, glycoprotein, glycolipid, lipid, carbohydrate, nucleic acid, and polysaccharide, as well as virus, fungus body, allergen, tissue and cell comprising any of them. Specific examples include a pollen-derived antigen, a hepatitis A virus-derived antigen, a hepatitis B virus-derived antigen, a hepatitis C virus-derived antigen, a hepatitis D virus-derived antigen, a hepatitis E virus-derived antigen, a hepatitis F virus-derived antigen, an HIV virus-derived antigen, an influenza virus-derived antigen, a herpesvirus (HSV-1, HSV-2)-derived antigen, an anthrax-derived antigen, a chlamydia-derived antigen, a pneumococcus-derived antigen, a Japanese encephalitis virus-derived antigen, a measles virus-derived antigen, a rubella virus-derived antigen, a Clostridium tetani-derived antigen, a varicella virus-derived antigen, a SARS virus-derived antigen, an EB virus-derived antigen, a papillomavirus-derived antigen, a Helicobacter pylori-derived antigen, a rabies virus-derived antigen, a West Nile virus-derived antigen, a hantavirus-derived antigen, a streptococcus-derived antigen, a staphylococcus-derived antigen, a Bordetella pertussis-derived antigen, a Mycobacterium tuberculosis-derived antigen, a Plasmodium-derived antigen, a poliomyelitis virus-derived antigen, various zoonotic infection-derived antigens, various food allergy-derived antigens, and a self-antigen.

Other preferable examples of the antigen include a cancer antigen. The cancer antigen is a substance derived from a protein specifically expressed in cancer cells, and is an antigen which is to be administered from the outside of a living boy to the inside of the living body to exert an effect of treatment and/or prevention of cancer by an immune response. The composition for enhancing immunogenicity of the present invention may be used as an active ingredient of a vaccine for treatment and/or prevention of cancer when the cancer antigen is used as the antigen. Accordingly, the present invention provides a vaccine containing the composition for enhancing immunogenicity of the present invention, as an active ingredient.

The composition for enhancing immunogenicity of the present invention can be used for treatment and/or prevention of disease such as cancer, infectious disease, or allergy. Examples of the cancer for which the composition for enhancing immunogenicity of the present invention may be used include basal cell carcinoma, Paget's disease, brain tumor, urinary bladder cancer, esophageal cancer, leukemia, lymphoma, liver cancer, gallbladder cancer, sarcoma, mastocytoma, adrenal cortex cancer, Ewing's tumor, Hodgkin's lymphoma, mesothelioma, multiple myeloma, thyroid cancer, skin cancer (for example, melanoma), lung cancer, pharynx cancer, gastric cancer, pancreatic cancer, colon cancer, kidney cancer, urinary bladder cancer, breast cancer, uterus cancer, ovarian cancer, prostate cancer, and head and neck cancer. Examples of the infectious disease includes an infectious disease by hepatitis A virus, hepatitis B virus, hepatitis C virus, hepatitis D virus, hepatitis E virus, hepatitis F virus, HIV virus, influenza virus, herpesvirus (HSV-1, HSV-2), anthrax, chlamydia, pneumococcus, Japanese encephalitis virus, measles virus, rubella virus, Clostridium tetani, varicella virus, SARS virus, EB virus, papillomavirus, Helicobacter pylori bacteria, rabies virus, West Nile virus, hantavirus, streptococcus, staphylococcus, Bordetella pertussis, Mycobacterium tuberculosis, Plasmodium, or poliomyelitis virus. Examples of the allergy include pollen allergy, various food allergies, and any allergy to a self-antigen.

The structure of the particle, which is one active ingredient of the composition for enhancing immunogenicity of the present invention and is composed of the amphiphilic polymer and the antigen, is not particularly limited. Since the particle is a complex of the amphiphilic polymer and the antigen, a structure in which the hydrophilic segment of the amphiphilic polymer constituting the particle is located inside the particle and the hydrophobic segment serves as an outer layer of the particle is preferable because it is formed the antigen to be enclosed inside the particle and may be more stably maintained.

The particle which is one active ingredient of the composition for enhancing immunogenicity of the present invention and is composed of the amphiphilic polymer and the antigen may comprise an additional component in addition to the antigen and the amphiphilic polymer. Specific examples of the additional component include well-known lipid as a component of a liposome or lipid nanoparticle, a particle capable of enclosing the antigen, such as a cancer antigen. However, as clear from Examples, the expected effects are sufficiently obtained in the present invention even without using any lipid in a component other than the antigen constituting the particle, and thus no lipid is preferably comprised as the additional component constituting the particle.

The method for producing the particle is not particularly limited, and examples thereof include a drying in liquid method, a spray-drying method, and a pulverizing method, and the particle in the present invention is preferably produced by a drying in liquid method.

Examples of the method for producing the particle by a drying method in liquid include an O/W emulsion method, a W/O/W emulsion method, and a S/O/W emulsion method.

The particle, when produced by an O/W emulsion method, may be produced by, for example, a step of preparing an O/W emulsion solution by mixing a water-immiscible organic solvent dissolving a powder of the amphiphilic polymer constituting the particle, and an aqueous solution dissolving a surface modifier and an antigen, and a step of obtaining the particle by removing the water-immiscible organic solvent from the O/W emulsion solution.

The particle, when produced by a W/O/W emulsion method, may be produced by, for example, a step of preparing a W/O emulsion solution by mixing an aqueous solvent dissolving an antigen and a water-immiscible organic solvent dissolving a powder of the amphiphilic polymer constituting the particle, a step of preparing a W/O/W emulsion solution by mixing the W/O emulsion solution and an aqueous surface modifier solution, and a step of obtaining the particle by removing the water-immiscible organic solvent from the W/O/W emulsion solution.

The particle, when produced by a S/O/W emulsion method, may be produced by, for example, a step of preparing a W/O emulsion solution by mixing an aqueous solvent dissolving an antigen and a water-immiscible organic solvent dissolving a powder of the amphiphilic polymer constituting the particle, a step of obtaining a solid content by removing the solvent from the W/O emulsion solution, a step of obtaining a S/O suspension solution by dispersing the solid content in the water-immiscible organic solvent, a step of preparing a S/O/W emulsion solution by mixing the S/O suspension solution and an aqueous surface modifier solution, and a step of obtaining the particle by removing the water-immiscible organic solvent from the S/O/W emulsion solution.

The content ratio of the antigen in the particle (antigen/particle) is preferably 0.01 to 20% by weight, more preferably 0.1 to 10% by weight, for example, 0.5 to 10% by weight, 0.5 to 8% by weight, 0.5 to 6% by weight, or 1 to 5% by weight. Examples of the method for quantitatively determining the content ratio of the antigen include a method comprising extracting the antigen from the particle using an organic solvent and quantifying the content ratio of the antigen by gel electrophoresis or liquid chromatography.

The surface modifier used for particle preparation is preferably a water-soluble polymer or a surfactant. The water-soluble polymer herein is a polymer compound having a solubility in water of 1 g (water-soluble polymer)/100 ml (water) or more.

Examples of the water-soluble polymer serving as the surface modifier include polyethylene glycol, polyvinylpyrrolidone, polyvinyl alcohol, polyethylenimine, polyacrylic acid, polymethacrylic acid, poly-1,3-dioxolane, 2-methacryloyloxyethyl phosphorylcholine polymer, poly-1,3,6-trioxane, polyamino acid, peptide, protein, sugar (monosaccharides, oligosaccharides, and polysaccharides), and preferably polyvinyl alcohol.

Examples of the surfactant serving as the surface modifier include a nonion activator such as polyoxyethylene polyoxypropylene glycol, sucrose fatty acid ester, polyoxyethylene fatty acid ester, polyoxyethylene sorbitan mono-fatty acid ester, polyoxyethylene sorbitan di-fatty acid ester, polyoxyethylene glycerin mono-fatty acid ester, polyoxyethylene glycerin di-fatty acid ester, polyglyceryl fatty acid ester, polyoxyethylene castor oil, or polyoxyethylene hydrogenated castor oil, alkyl sulfate such as sodium lauryl sulfate, ammonium lauryl sulfate, or sodium stearyl sulfate, or lecithin, and preferably polyoxyethylene polyoxypropylene glycol.

The water-immiscible organic solvent used in particle preparation preferably can dissolve the amphiphilic polymer and hardly dissolves or does not dissolve β-glucan. The solubility in water of the water-immiscible organic solvent is preferably 30 g (water-immiscible organic solvent)/100 ml (water) or less. Specific examples of the water-immiscible organic solvent include ethyl acetate, isopropyl acetate, butyl acetate, dimethyl carbonate, diethyl carbonate, methylene chloride, and chloroform.

The aqueous solvent used in particle preparation is an aqueous solution containing water and optionally a water-soluble component. Examples of the water-soluble component include inorganic salts, sugars, organic bases, amino acids, peptides, proteins, and nucleic acids.

The surface modifier used in the above production process may be bound to the particle surface. The bond herein may be a non-covalent bond or a covalent bond. The non-covalent bond is preferably hydrophobic interaction, and may be ion bond (electrostatic interaction), hydrogen bond, coordinate bond, van der Waals binding, or physical adsorption, or may be a combination thereof.

The average particle size of the particle is preferably 0.1 to 50 µm, more preferably 0.1 to 25 µm, further preferably 0.1 to 10 µm, particularly preferably 0.1 to 1 µm, for example, 0.2 to 1 µm, 0.3 to 1 µm, 0.3 to 0.8 µm, 0.3 to 0.7 µm, or 0.3 to 0.6 µm. The average particle size herein may be determined with a dynamic light scattering apparatus (DLS: for example, ELS-Z, OTSUKA ELECTRONICS CO., LTD) according to a cumulant method.

The immuno-stimulating factor as one active ingredient of the composition for enhancing immunogenicity of the present invention is described. The term "immuno-stimulating factor" herein means a substance which is a factor activating one or more immune cells and maintaining and/or enhancing the immune function of such cells. The term "immune cells" herein include, for example, T lymphocytes, B lymphocytes, NK cells, monocytes, dendritic cells, granulocytes, macrophages, bone marrow-derived inhibitory cells, Langerhance cells and a group of precursor cells thereof, and a group of the immune cells in tumors.

The immuno-stimulating factor for use in the present invention is not particularly limited, and specific examples thereof include a ligand or agonist binding to a Toll-like receptor (TLR), a NOD-like receptor (NLR), a RIG-like receptor, a C-type lectin receptor (CLR) or a stimulator of interferon gene (STING), and preferably a ligand or agonist binding to TLR.

Specific examples of TLR include TLR2, TLR3, TLR4, TLR5, TLR7, TLR8, TLR9 or TLR11, and specific examples of the ligand or agonist binding thereto include the following (i) to (vii):
(i) a ligand or agonist binding to TLR2 selected from the group consisting of peptidoglycan, lipoprotein, lipopolysaccharide, and zymosan.
(ii) a ligand or agonist binding to TLR3 selected from the group consisting of poly(I:C) and poly(A:U).
(iii) a ligand or agonist binding to TLR4 selected from the group consisting of lipopolysaccharide (LPS), HSP60, RS09, and MPLA.
(iv) flagellin as a ligand or agonist binding to TLR5.
(v) a ligand or agonist binding to TLR7 or 8 selected from the group consisting of an imidazoquinoline compound and single-strand RNA.
(vi) a ligand or agonist binding to TLR9 selected from the group consisting of bacterial DNA, unmethylated CpG DNA, hemozorin, ODN1585, ODN1668 and ODN1826.
(vii) a ligand or agonist binding to TLR11 selected from the group consisting of profilin and uropathogenic bacteria.

The immuno-stimulating factor in the present invention is preferably a ligand or agonist binding to TLR3, TLR7 or 8 (TLR3, TLR7/8 ligand or agonist), more preferably the ligand or agonist of the above item (ii) or (v).

Regarding the above item (ii) above, which is a ligand or agonist binding to TLR3 selected from the group consisting of poly(I:C) and poly(A:U), poly(I:C) is preferred. Poly(I:C) is double-stranded RNA formed by an RNA chain only comprising inosine as a base and an RNA chain only comprising cytidine as a base. The chain length of poly(I:C) used in the present invention is not particularly limited.

Preferable specific examples of the imidazoquinoline compound in above item (v) include any compound described in US. Patent No. 8,951,528 and any compound described in WO2015/103989, and examples thereof include 4-amino-2-(ethoxymethyl)-a,a-dimethyl-1H-imidazo[4,5-c]quinoline-1-ethanol (Resiquimod (R848)), 1-(2-methylpropyl)-1H-imidazo[4,5-c]quinoline-4-amine (Imiquimod), 1-(4-amino-2-ethylaminomethylimidazo-[4,5-c]quinolin-1-yl)-2-methylpropan-2-ol (Gardiquimod), N-[4-(4-amino-2-ethyl-1H-imidazo[4,5-c]quinolin-1-yl)butyl-]methanesulfonamide (PF-4878691), 4-amino-aa-dimethyl-2-methoxyethyl-1H-imidazo[4,5-c]quinoline-1-ethanol, 1-(2-(3-(benzyloxy)propoxy)ethyl)-2-(ethoxymethyl)-1H-imidazo[4,5-c]quinolin-4-amine, 4-amino-2-ethoxymethyl-aa-dimethyl-6,7,8,9-tetrahydro-1H-imidazo[4,5-c]quinoline-1-ethanol, N-(2-{2-[4-amino-2-(2-methoxyethyl)-1H-imidazo[4,5-c]quinolin-1-yl]eyhoxy}ethyl)-n'-phenylurea, 1-2-amino-2-methylpropyl)-2-(ethoxymethyl)-1H-imidazo[4,5 -c]quinolin-4-amine, 1-{4-[(3,5-dichlorophenyl)sulfonyl]butyl}-2-ethyl-1H-imidazo[4,5-c]quinolin-4-amine, N-(2-{2-[4-amino-2-(ethoxymethyl)-1H-imidazo[4,5-c]quinolin-1-yl]ethoxy}ethyl)-n'-cyclohexylurea, N-{3-[4-amino-2-(ethoxymethyl)-1H-imidazo[4,5-c]quinolin-1-yl]propyl}-n'-(3-cyanophenyl)thiourea, N-[3-(4-amino-2-butyl-1H-imidazo[4,5-c]quinolin-1-yl)-2,2-dimethylpropyl]benzamide, 2-butyl-1-[3-(methylsulfonyl)propyl]-1H-imidazo[4,5-c]quinolin-4-amine, and derivatives thereof, but the imidazoquinoline compound is not particularly limited as long as it can bind to TLR7 or TLR8.

Specific examples of the ligand or agonist binding to a NOD-like receptor (NLR) include M-TriDAP and PGN, as well as the ligand or agonist binding to NOD1 such as Tri-DAP, iE-DAP, and C12-iE, and the ligand or agonist binding to NOD2 such as MDP, N-glycosyl-MDP, Murabutide, M-TriLyS-D-ASN, M-TriLYS, and L18-MDP.

Specific examples of the ligand or agonist binding to a RIG-like receptor include 5'ppp-dsRNA, poly(dA:dT), poly(dG:dC), and poly(I:C).

Specific examples of the ligand or agonist binding to a C-type lectin receptor (CLR) include trehalose 6,6-dibehenate, zymosan, WGP, HKSC, HKCA, and curdlan AL.

Specific examples of the ligand or agonist binding to a stimulator of interferon gene (STING) include c-di-GMP, c-di-AMP, 2'3'-cGAMP, 3'3'-cGAMP or 2'2'-cGAMP.

In a mixture of the particle and the immuno-stimulating factor, which is an active ingredient of the composition for enhancing immunogenicity of the present invention, each of the particle and the immuno-stimulating factor may be comprised in an independent state (namely, the immuno-stimulating factor may be comprised as a separate structure from the particle), or the immuno-stimulating factor may be comprised in a state of being enclosed in the particle (namely, as a mixed particle), and preferably, a mixture comprising each of the particle and the immuno-stimulating factor in an independent state.

The method for producing a mixture comprising each of the particle and the immuno-stimulating factor in an independent state is not particularly limited, and for example, the mixture is produced by dispersing the particle produced by the production method described above, in an aqueous solvent, and adding and mixing a dissolved liquid of the immuno-stimulating factor. In this case, treatments such as particle preparation and formation of crosslink by chemical bond are not performed after the mixing. The method for producing a mixture in which the immuno-stimulating factor is enclosed in the particle is not particularly limited, and the mixture is produced by using a solution mixture of an antigen/immuno-stimulating factor, in which an aqueous solvent dissolving the antigen, which is prepared in the particle production step, is mixed with a dissolved liquid of the immuno-stimulating factor, or an aqueous solution of an antigen-immuno-stimulating factor complex in which the antigen is modified with the immuno-stimulating factor by chemical bonding, or by mixing the immuno-stimulating factor with a water-immiscible organic solvent dissolving a powder of the amphiphilic polymer constituting the particle.

The method for producing a mixture in which the immuno-stimulating factor is enclosed in the particle is not particularly limited, and the mixture may be produced by the following (1) or (2), depending on whether the immuno-stimulating factor has hydrophilicity or hydrophobicity.

### (1) Production method when immuno-stimulating factor has hydrophilicity

While an aqueous solution mixture of an antigen/immuno-stimulating factor in which an aqueous solvent dissolving the antigen is mixed with an aqueous solution of the immuno-stimulating factor may be used, the mixture is preferably produced by using an aqueous solution of an antigen-immuno-stimulating factor complex, produced by separately chemically bonding the immuno-stimulating factor to the antigen. Examples include a production method by mixing a hydrophobic complex formed by ion bonding between a chargeable immuno-stimulating factor and a polymer additive having opposite charge thereto, with a water-immiscible organic solvent dissolving a powder of the amphiphilic polymer constituting the particle, and physically adsorbing to a hydrophobic segment of the particle.

### (2) Production method when immuno-stimulating factor has hydrophobicity

Examples include a production method using an aqueous solution of an antigen-immuno-stimulating factor complex, produced by separately chemically bonding an immuno-stimulating factor which does not inhibit hydrophilicity of the antigen, or a production method by mixing the immuno-stimulating factor with a water-immiscible organic solvent dissolving a powder of the amphiphilic polymer constituting the particle, and physically adsorbing to a hydrophobic segment of the particle.

The weight ratio between the particle and the immuno-stimulating factor, active ingredients of the composition for enhancing immunogenicity of the present invention, is not particularly limited as long as the objects of the present invention are achieved, and the ratio of the weight of the immuno-stimulating factor to the weight of the particle is preferably 0.001 to 10000% by weight, more preferably 0.01 to 1000% by weight, further preferably 0.1 to 500% by weight, for example, 0.5 to 400% by weight, 1.0 to 300% by weight, 1.2 to 200% by weight, 1.4 to 150% by weight, or 1.5 to 100% by weight.

According to a preferred aspect of the present invention, an immuno-stimulating method (in particular, method for enhancing immunogenicity) which comprises administering the composition for enhancing immunogenicity, the medicine, or the vaccine of the present invention, to a (organism) subject is provided. A method for treating and/or preventing a disease such as cancer, infectious disease , or allergy which comprises administering the composition for enhancing immunogenicity, the medicine, or the vaccine of the present invention, to a (organism) subject is also provided. The method for activating immune reaction and the method for treating and/or preventing a disease, using the composition for enhancing immunogenicity, the medicine, or the vaccine of the present invention, are not limited. The composition for enhancing immunogenicity, the medicine, or the vaccine may be administered to an organism (subject), or may be contacted with immune competent cells harvested out of an organism (subject). The method for administration to an organism (subject) is not particularly limited, and examples thereof include subcutaneous administration, intracutaneous administration, intramuscular administration, intranasal administration, transpulmonary administration, oral administration, percutaneous administration, sublingual administration, intravaginal administration, intraperitoneal administration, and lymph node administration, and preferably, intracutaneous administration or subcutaneous administration. The organism (subject) for administration may be human or any animal other than human, and is preferably human, or pig, cow, bird, sheep, horse, donkey, goat, camel, dog, cat, ferret, rabbit, monkey, rat, mouse or guinea pig being kept as a livestock animal, a pet animal or a laboratory animal. The subject may be a subject having a disease such as cancer, infectious disease, or allergy, or may be a subject without a disease such as cancer, infectious disease, or allergy, but having a risk of developing the disease.

In a case where the composition for enhancing immunogenicity of the present invention is used as a medicine (including a vaccine), various pharmaceutically useful additives may be compounded to formulate the medicine, and specific examples of such an additive include a buffering agent, an antioxidant, a salt, a polymer, or sugar.

The dose in the case of using the composition for enhancing immunogenicity of the present invention as a medicine is appropriately set depending on the administration method and the frequency of administration. For example, in a case where the composition for enhancing immunogenicity of the present invention is subcutaneously administered to human, 0.01 to 1,000 mg of the composition is administered per administration.

### Examples

Examples are shown below, but the present invention is not limited by these Examples.

### (Example 1) Synthesis of black yeast glucan-poly(lactic-co-glycolic acid)

In the present Example, poly(lactic-co-glycolic acid) (PLGA) was added to black yeast glucan to perform condensation reaction, thereby synthesizing a straight-chained block-type polymer.

### <Synthesis of black yeast glucan with a primary amino group at reducing terminal (black yeast glucan-NH₂)>

After 5 g of black yeast glucan (DAISO CO.,LTD.) was dissolved in 150 ml of dimethylsulfoxide, 5 ml of an aqueous 35% hydrochloric acid solution was added, and the resultant was stirred at 105°C for 20 minutes. The reaction solution was transferred to a dialysis membrane, and dialysis was performed in water, followed by freeze-drying to obtain a black yeast glucan hydrolysate (number-average molecular weight 2,400) as a powder.

Sodium triacetoxyborohydride (413.3 mg) and N-Boc-ethylenediamine (246.9 µL) were loaded into a dimethylsulfoxide solution of a black yeast glucan hydrolysate (number-average molecular weight 2,400) having a concentration of 2.5 g/8 ml, and then reacted with stirring at 55°C for 169.5 hours. Next, unreacted N-Boc-ethylenediamine was removed by dialysis with water as an external solution, and black yeast glucan-NHBoc was obtained. Deprotection reaction of a Boc terminal group of the resulting black yeast glucan-NHBoc (1.19 g) (aqueous 35% hydrochloric acid solution (14.3 ml)/dimethylsulfoxide (24 ml), stirred at ambient temperature for 2 hours) was performed, and then purification by water dialysis was performed, and a polymer powder (black yeast glucan-NH₂) was obtained by freeze-drying.

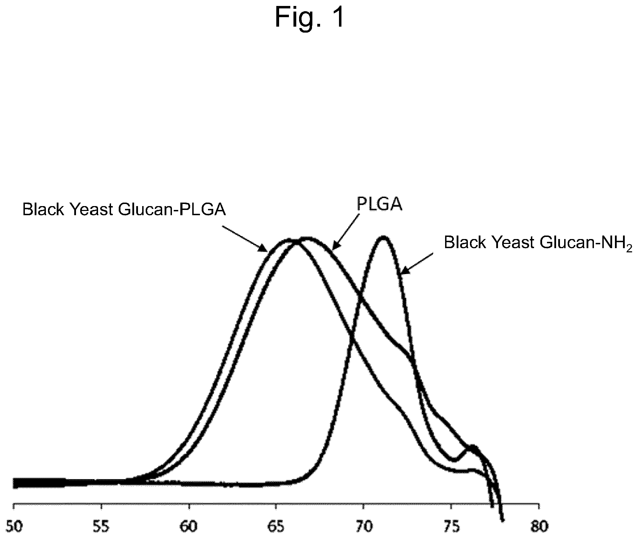

The number-average molecular weight of the black yeast glucan-NH₂ was determined by GPC measurement (column: TSK-gel α-5000 × 2 manufactured by Tosoh Corporation, DMF-based solvent, detector: RI, standard product: pullulan) (Figure 1: black yeast glucan-NH₂). It was confirmed by ¹H-NMR measurement that a primary amino group was introduced into a reducing terminal of black yeast glucan.

### <Synthesis of black yeast glucan-PLGA>

Poly(lactic-co-glycolic acid) having a heterobifunctional terminal group (COOH-PLGA-OH, FUJIFILM Wako Pure Chemical Corporation, PLGA-5020, number-average molecular weight 8,900) (4.462 g) was mixed in a dimethylsulfoxide (2.5 ml) solution of 1-ethyl-3-(3-dimethylaminopropyl)carbodiimide hydrochloride (EDC) (479.25 mg) and N-hydroxysuccinimide (NHS) (287.73 mg), and then reacted at 50°C for approximately 18 hours, thereby converting a carboxyl group at the α-terminal into an active ester (NHS). The reaction solution was loaded with black yeast glucan having a primary amino group at the ω-terminal (black yeast glucan-NH₂, number-average molecular weight 1,500) (749 mg), and condensation reaction (270.5 hours) of an active ester (NHS) of NHS-PLGA-OH and the primary amino group of black yeast glucan-NH₂ was performed. After the reaction, unreacted black yeast glucan-NH₂ was removed by water dialysis, and then unreacted NHS-PLGA-OH was removed by ultra-centrifugal purification to obtain a black yeast glucan-PLGA polymer 1.

Black yeast glucan-NH₂ having number-average molecular weights of 1,200 and 2,300 were synthesized according to the above synthesis method of black yeast glucan-NH₂, and a black yeast glucan-PLGA polymer 2 was obtained by using black yeast glucan-NH₂ (number-average molecular weight 1,200) andthePLGA-5020 (number-average molecular weight 8,900), and a black yeast glucan-PLGA polymer 3 was obtained using black yeast glucan-NH₂ (number-average molecular weight 2,300) and the PLGA-5020 (number-average molecular weight 8,900) according to the same synthesis method as for the black yeast glucan-PLGA polymer 1.

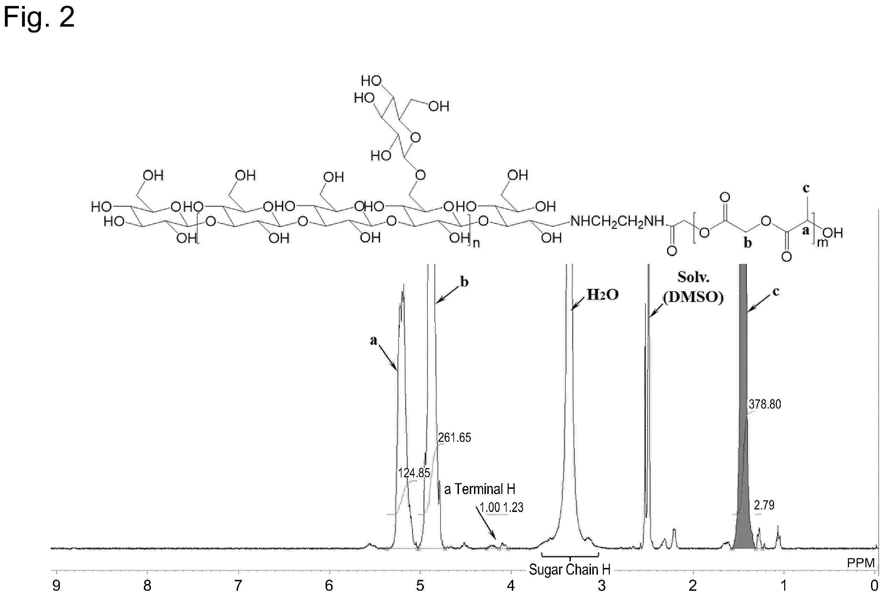

The number-average molecular weight of black yeast glucan-PLGA was determined by GPC measurement (column: TSK-gel α-5000 × 2, manufactured by Tosoh Corporation, DMF-based solvent, detector: RI, standard product: pullulan) (Table 1, Figure 1: black yeast glucan-PLGA). It was confirmed by ¹H-NMR measurement that condensation reaction progressed (Figure 2: black yeast glucan-PLGA).

**[Table 1]**

| Results of analysis on black yeast glucan-PLGA (1, 2, 3) | | | | |
|---|---|---|---|---|
| Amphiphilic polymer | Number-average molecular weight | Hydrophilic polysaccharide portion | Number-average molecular weight of hydrophilic polysaccharide | Number-average molecular weight of PLGA |
| 1 | 12200 | Black yeast glucan | 1500 | 10700 |
| 2 | 12400 | Black yeast glucan | 1200 | 11200 |
| 3 | 9900 | Black yeast glucan | 2300 | 7600 |

### (Comparative Example 1) Synthesis of dextran-poly(lactic-co-glycolic acid)

In this Comparative Example, poly(lactic-co-glycolic acid) (PLGA) was added to dextran to perform condensation reaction, thereby synthesizing a straight-chained block-type polymer.

### <Synthesis of dextran with a primary amino group at reducing terminal (dextran-NH₂)>

Sodium triacetoxyborohydride (203.5 mg) and N-Boc-ethylenediamine (76.1 µL) were loaded into a dimethylsulfoxide solution of dextran (number-average molecular weight 2,100, Pharmacosmos A/S) having a concentration of 500 mg/2 ml, and then reacted with stirring at 60°C for 91 hours. Next, unreacted N-Boc-ethylenediamine was removed by dialysis with water as an external solution, freeze-drying was performed, and dextran-NHBoc was synthesized. Deprotection reaction of a Boc terminal group of the resulting dextran-NHBoc (450 mg) (aqueous 35% hydrochloric acid solution (5 ml)/dimethylsulfoxide (5 ml), stirred at ordinary temperature for 35 hours) was performed, and then purification by water dialysis was performed, and a polymer powder (dextran-NH₂) was obtained by freeze-drying.

The number-average molecular weight of the dextran-NH₂ was determined by GPC measurement (column: TSK-gel α-5000 × 2, manufactured by Tosoh Corporation, DMF-based solvent, detector: RI, standard product: pullulan). It was confirmed by ¹H-NMR measurement that a primary amino group was introduced to a reducing terminal of dextran.

### <Synthesis of dextran-PLGA>

Poly(lactic-co-glycolic acid) having a heterobifunctional terminal group (COOH-PLGA-OH, FUJIFILM Wako Pure Chemical Corporation, PLGA-5020, number-average molecular weight 8,900) (350 mg) was mixed in a dimethylsulfoxide (0.58 ml) solution of EDC (35.5 mg) and NHS (21.3 mg), and then reacted at 50°C for 2 hours, thereby converting a carboxyl group at one terminal into an active ester (NHS). The reaction solution, dextran to which a primary amino group was introduced at a reducing terminal (dextran-NH₂, number-average molecular weight 2,700) (100 mg) was added, and condensation reaction of an active ester (NHS) of NHS-PLGA-OH and the primary amino group of dextran-NH₂ was performed for 110.5 hours. After the reaction, unreacted dextran-NH₂ was removed by water dialysis, and then unreacted NHS-PLGA-OH was removed by ultra-centrifugal purification to obtain a dextran-PLGA polymer 4.

A dextran-PLGA polymer 5 was obtained by the same method as for the dextran-PLGA polymer 4, using dextran-NH₂ (number-average molecular weight 1,800) and the PLGA-5020 (number-average molecular weight 8,900).

The number-average molecular weight of dextran-PLGA was determined by GPC measurement (column: TSK-gel α-5000 × 2, manufactured by Tosoh Corporation, DMF-based solvent, detector: RI, standard product: pullulan) (Table 2: dextran-PLGA). It was confirmed by ¹H-NMR measurement that condensation reaction progressed.

**[Table 2]**

| Results of analysis on dextran-PLGA (4, 5) | | | | |
|---|---|---|---|---|
| Amphiphilic polymer | Number-average molecular weight | Hydrophilic polysaccharide portion | Number-average molecular weight of hydrophilic polysaccharide | Number-average molecular weight of PLGA |
| 4 | 14500 | Dextran | 2700 | 11800 |
| 5 | 13400 | Dextran | 1800 | 11600 |

### (Example 2) Preparation of particles (OVA-containing black yeast glucan particle (1) and comparative particle: OVA-containing dextran particle (2)), by S/O/W emulsion method

The polymer powder (50 mg) of the black yeast glucan-PLGA polymer (Table 1, amphiphilic polymer 1) was dissolved in a mixed solution of 0.9 ml of dimethyl carbonate and 100 µl of tert-butanol to prepare a polymer solution. After dripping 0.5 ml of an aqueous 0.5% (w/v) OVA (ovalbumin, Sigma-Aldrich Co. LLC) solution into the polymer solution, the resultant was stirred with a mixer (PT2100S manufactured by Polytron) at 11,000 rpm for 1 minute to produce a W/O emulsion solution. The W/O emulsion solution was preliminarily frozen by liquid nitrogen, and then freeze-dried with a freeze-dryer (TOKYO RIKAKIKAI CO., LTD., FD-1000) at a trap cooling temperature of -45°C and a degree of vacuum of 20 Pa for 12 hours. The resulting solid content was dispersed in 5 ml of ethyl acetate to prepare a S/O suspension solution. The S/O suspension solution was dripped in 20 ml of an aqueous 1% (w/v) polyvinyl alcohol solution, and stirred with a mixer (Silverson Nippon Limited, L5M-A) at 6,000 rpm for 5 minutes to prepare a S/O/W-type emulsion solution. Ethyl acetate was removed from the S/O/W-type emulsion solution by drying in liquid, and a particle suspension liquid was obtained. The suspension liquid was transferred to a 50-ml tube, and a particle was precipitated by centrifugation at 8,000 rpm for 10 minutes. After the supernatant was removed, the particle was re-suspended in 25 ml of distilled water, and washed by re-precipitation of the particle with centrifugation in the above conditions. After the washing operation was repeated once more , and removing the supernatant, the particle was suspended in 4 ml of an aqueous solution containing 5% (w/v) mannitol and 0.1% (w/v) polysorbate 80. The suspension liquid was preliminarily frozen by liquid nitrogen and then freeze-dried with a freeze-dryer at a trap cooling temperature of -45°C and a degree of vacuum of 20 Pa for 12 hours to obtain an OVA-containing black yeast glucan particle (1).

An OVA-containing dextran particle (2) was obtained as a comparative particle by the same method as for the above OVA-containing black yeast glucan particle, using a dextran-PLGA polymer (Table 2, amphiphilic polymer 4).

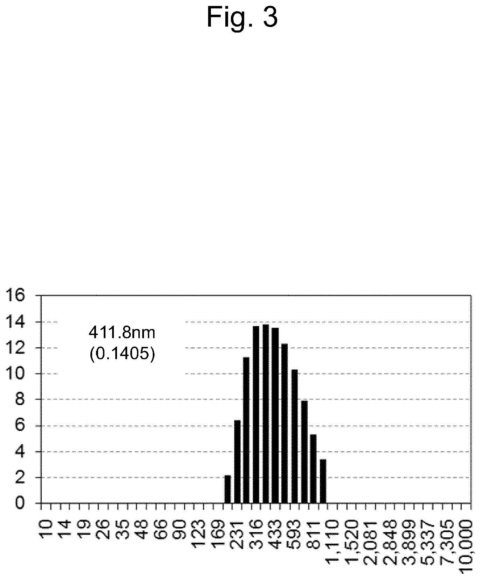

The results of evaluation for each particle are shown in Table 3. The average particle size of the particle was calculated by a cumulant method using a dynamic light scattering apparatus (ELS-Z manufactured by OTSUKA ELECTRONICS CO., LTD.). The results of dynamic light scattering (DLS) measurement of particle (1) are shown in Figure 3. The content ratio (w/w) of the OVA antigen was determined by extracting the antigen from the particle using an organic solvent, subjecting the extracted antigen to gel electrophoresis using a gel electrophoresis apparatus (TEFCO) and then staining with a colloidal CBB staining kit (TEFCO).

**[Table 3]**

| Results of analysis on particles (OVA-containing black yeast glucan particle (1) and comparative particle (2): OVA-containing dextran particle (2)) prepared by S/O/W emulsion method | | | |
|---|---|---|---|
| Particle | Substrate polymer | Average particle size (nm) | Antigen content ratio (%) |
| (1) | Black yeast glucan-PLGA | 411.8 | 4.47 |
| Comparative particle (2) | Dextran-PLGA | 629.7 | 3.49 |

### (Reference Example 1) Induction of mouse bone marrow-derived dendritic cell

C57BL/6 female mice (manufactured by Japan SLC, Inc.) were each euthanized by cervical dislocation, and then the femur was collected. Both ends of the femur were cut by scissors, a RPMI1640 medium (hereinafter, RPMI medium) containing 10% fetal bovine serum (FBS, Sigma-Aldrich Co. LLC), 100 units/ml of penicillin, and 100 µg/ml of streptomycin (NACALAI TESQUE, INC.) was injected into the femur with an injector, and bone-marrow cells were collected. The cells were precipitated by centrifugation at 410 g for 5 minutes to collect the supernatant. The cells collected were suspended in 1 ml of a buffer for hemolysis (BD Bioscience), and then left to stand at room temperature for 5 minutes and hemolyzed. 10 ml of the RPMI medium was added to a cell suspension liquid after hemolysis, the cells were precipitated by centrifugation at 1,500 rpm for 5 minutes, and the supernatant was removed. The cells were suspended in a RPMI medium (hereinafter, culture medium) containing GM-CSF, and then seeded to a 6-well plate (Corning Incorporated, Flat Bottom, Tissue Culture-Treated, Polystyrene). The plate to which the cells were seeded was incubated in a CO₂ incubator (PHC Corporation) set to conditions of 5% CO₂, 37°C, and a humidity of 100%. The culture medium was exchanged on day 2 and day 5, and floating cells were aspirated on day 6 of the culturing and then strongly suspended with a micropipette, thereby collecting cells slightly bound to the plate, namely, only induced dendritic cells. The dendritic cells collected were suspended in "CELLBANKER2" (NIPPON ZENYAKU KOGYO CO., LTD.) and stored at - 150°C until use.

### (Example 3) Activation of mouse bone marrow-derived dendritic cells by composition for enhancing immunogenicity 1

### <Method>

The dendritic cells obtained in Reference Example 1 were seeded at 1 × 10⁵ per well to a 96-well plate together with a culture medium, and then a mixture, in which each component was comprised in an independent state, prepared by mixing the OVA-containing black yeast glucan particle (1) obtained in Example 2 with poly(I:C) (Sigma-Aldrich Co. LLC, catalog number: P1530) as an immuno-stimulating factor was added with a particle concentration (except for mannitol) of 20 µg/ml and a poly(I:C) concentration of 20 µg/ml. For comparison, only the OVA-containing black yeast glucan particle (1) was added with a particle concentration (except for mannitol) of 20 µg/ml. The plate to which the cells were seeded was incubated in a CO₂ incubator for 18 hours. After removing the supernatant, 0.5 mM EDTA was added and incubated for 5 minutes to exfoliate the cells, and the cells were collected with a micropipette. A cell suspension liquid collected was replaced to be in a phosphate buffer solution containing 1% FBS, and the antibody labeling reaction was performed by adding an APC-labeled anti-CD86 antibody, a FITC-labeled anti-CD11c antibody and an APC-Cy7-labeled NMCII antibody, and being left to stand at 4°C for 30 minutes. After terminating the antibody labeling reaction, the expression level of an activation marker (CD86) of dendritic cells (CD11c-positive MHCII-positive cells) was evaluated by the mean fluorescence intensity (MFI) using flow cytometry.

Regarding a mixture comprising each of the OVA-containing dextran particle (2) and poly(I:C) (Sigma-Aldrich Co. LLC, catalog number: P1530) in an independent state, and only the OVA-containing dextran particle (2), as Comparative Examples, the respective expression levels of activation markers were evaluated similarly by MFI.

### <Results>

The MFI of CD86 for the mixture of the OVA-containing black yeast glucan particle (1) and poly(I:C) was increased 4.9 times compared to that in only the OVA-containing black yeast glucan particle (1), and the MFI of CD86 in the mixture of the OVA-containing dextran particle (2) and poly(I:C) was increased 2.1 times compared to that in only the OVA-containing dextran particle (2) (Table 4).

According to the left graph in Figure 9 of WO2016/199904, there is no significant difference between the immuno-stimulating capacity (evaluated with antigen-specific serum total IgG) by the muscular injection of a mixture of an antigen (OVA), curdlan as one β-glucan, and other immuno-stimulating factor (CpG ODN) into mice, and the immuno-stimulating capacity with a mixture of an antigen (OVA) and β-glucan (curdlan). It was thus expected that the immuno-stimulating capacity with a mixture of the OVA-containing black yeast glucan particle (1) (constituted from OVA and β-glucan (black yeast glucan)-PLGA) and an immune activator was comparable with the immuno-stimulating capacity with only the OVA-containing black yeast glucan particle (1). However, as shown in the present Example, it was found that the MFI of CD86 for the mixture of the OVA-containing black yeast glucan particle (1) and poly(I:C) was increased 4.9 times compared to that in only the OVA-containing black yeast glucan particle (1) (group of no addition of poly(I:C)), and that unexpectedly high immuno-stimulating capacity was realized. It was also unexpected that the rate of increase in MFI for a mixture with poly(I:C) relative to that for a group without the addition of poly(I:C) was remarkably enhanced by the use of the OVA-containing black yeast glucan particle (1), since it was assumed that the rate of increase in MFI for a mixture using the OVA-containing black yeast glucan particle (1) relative to that for a group without the addition of poly(I:C), and the rate of increase in MFI for a mixture using the OVA-containing dextran particle (2) relative to that for a group without the addition of poly(I:C) were at the same level.

**[Table 4]**

| Activation of mouse bone marrow-derived dendritic cells by mixture of OVA-containing black yeast glucan particle (1) or comparative particle (2): OVA-containing dextran particle (2)) and poly(I:C) | | |
|---|---|---|
| | Expression intensity of CD86 (MFI) | Rate of increase relative to group without poly(I:C) |
| Particle (1) + poly(I:C) | 2065 | 4.9 times |
| Comparative particle (2) + poly(I:C) | 1022 | 2.1 times |

### (Example 4) Activation of mouse bone marrow-derived dendritic cells with composition for enhancing immunogenicity 2

### <Method>

Evaluation was performed by the same method as in Example 3, by using the dendritic cells obtained in Reference Example 1. A mixture prepared by mixing the OVA-containing black yeast glucan particle (1) obtained in Example 2 and Resiquimod (Resiquimod (R848); Sigma-Aldrich Co. LLC, catalog number: SML0196) being an imidazoquinoline compound and known as a TLR7/8 agonist, as the immuno-stimulating factor, in which each component was comprised in an independent state, was added with a particle concentration (except for mannitol) of 20 µg/ml and with an R848 concentration of 0.3 µg/ml. For comparison, only the OVA-containing black yeast glucan particle (1) was added with a particle concentration (except for mannitol) of 20 µg/ml.

### <Results>

The MFI of CD86 for the mixture of the OVA-containing black yeast glucan particle (1) and R848 was increased 1.4 times compared to that for only the OVA-containing black yeast glucan particle (1) (Table 5). According to the left graph in Figure 13 of WO2016/199904, there was no significant difference between the immuno-stimulating capacity (evaluated with antigen-specific serum total IgG) by the muscular injection of a mixture of an antigen (OVA), curdlan as one β-glucan and other immuno-stimulating factor (R848) into mice, and the immuno-stimulating capacity with a mixture of an antigen (OVA) and R848, and thus the immuno-stimulating capacity with the mixture of the OVA-containing black yeast glucan particle (1) and R848 was expected to be comparable with the immuno-stimulating capacity with only the OVA-containing black yeast glucan particle (1). Therefore, it was unexpected that the mixture of the OVA-containing black yeast glucan particle (1) and the immuno-stimulating factor exhibited remarkable immuno-stimulating capacity, as in Example 3.

**[Table 5]**

| Activation of mouse bone marrow-derived dendritic cells by mixture of OVA-containing black yeast glucan particle (1) and R848 | | |
|---|---|---|
| | Expression intensity of CD86 (MFI) | Rate of increase relative to group without R848 |
| Particle (1) + R848 | 596 | 1.4 times |

### Industrial Applicability

The composition for enhancing immunogenicity of the present invention can be used in a medicine, particularly in a vaccine for treatment and/or prevention of infectious disease, cancer, or the like.

All publications, patents, and patent applications herein cited are to be herein incorporated by reference as they are.

## Claims

1. A composition for enhancing immunogenicity, containing, as an active ingredient, a mixture of a particle and an immuno-stimulating factor, wherein the particle comprises an amphiphilic polymer in which a hydrophobic segment is poly(hydroxy acid) and a hydrophilic segment is β-glucan, and an antigen.

2. The composition for enhancing immunogenicity according to claim 1, wherein the mixture comprises each of the particle and the immuno-stimulating factor in an independent state.

3. The composition for enhancing immunogenicity according to claim 1 or 2, wherein the β-glucan is a polymer of glucose linked by one or more β-1,3 bonds and/or one or more β-1,6 bonds.

4. The composition for enhancing immunogenicity according to any one of claims 1 to 3, wherein the β-glucan is black yeast glucan, curdlan, pachyman, laminaran, lichenan, schizophyllan, lentinan, scleroglucan, or pachymaran.

5. The composition for enhancing immunogenicity according to any one of claims 1 to 4, wherein the poly(hydroxy acid) is poly(lactic-co-glycolic acid), polylactic acid, or polyglycolic acid.

6. The composition for enhancing immunogenicity according to any one of claims 1 to 5, wherein the immuno-stimulating factor is a ligand or agonist binding to a Toll-like receptor (TLR), a NOD-like receptor (NLR), a RIG-like receptor or a C-type lectin receptor (CLR), or a stimulator of interferon gene (STING).

7. The composition for enhancing immunogenicity according to claim 6, wherein the ligand or agonist binding to the Toll-like receptor (TLR) is a ligand or agonist binding to TLR2, TLR3, TLR4, TLR5, TLR7, TLR8, TLR9, or TLR11.

8. The composition for enhancing immunogenicity according to claim 6 or 7, wherein the ligand or agonist binding to the Toll-like receptor (TLR) is any of the following (i) to (vii):
(i) a ligand or agonist binding to TLR2 selected from the group consisting of peptidoglycan, lipoprotein, lipopolysaccharide, and zymosan;
(ii) a ligand or agonist binding to TLR3 selected from the group consisting of poly(I:C) and poly(A:U);
(iii) a ligand or agonist binding to TLR4 selected from the group consisting of lipopolysaccharide (LPS), HSP60, RS09, and MPLA;
(iv) flagellin as a ligand or agonist binding to TLR5;
(v) a ligand or agonist binding to TLR7 or 8 selected from the group consisting of an imidazoquinoline compound and single-strand RNA;
(vi) a ligand or agonist binding to TLR9 selected from the group consisting of bacterial DNA, unmethylated CpG DNA, hemozorin, ODN1585, ODN1668, and ODN1826; and
(vii) a ligand or agonist binding to TLR11 selected from the group consisting of profilin and uropathogenic bacteria.

9. The composition for enhancing immunogenicity according to any one of claims 1 to 8, wherein a number-average molecular weight of the amphiphilic polymer is 500 to 100,000 (molar ratio).

10. The composition for enhancing immunogenicity according to any one of claims 1 to 9, wherein an average particle size of the particle is 0.1 to 50 µm.

11. A medicine containing the composition for enhancing immunogenicity according to any one of claims 1 to 10, as an active ingredient.

12. A vaccine containing the composition for enhancing immunogenicity according to any one of claims 1 to 10, as an active ingredient.

13. A vaccine for treatment and/or prevention of cancer, containing the composition for enhancing immunogenicity according to any one of claims 1 to 10, as an active ingredient.

14. A method for enhancing immunogenicity, comprising administering the composition for enhancing immunogenicity according to any one of claims 1 to 10, to a subject.

15. A method for treating and/or preventing cancer, comprising administering the composition for enhancing immunogenicity according to any one of claims 1 to 10, the medicine according to claim 11, or the vaccine according to claim 12 or 13, to a subject.
